# EUROPEAN PATENT APPLICATION

(11) **EP 4 285 973 A1**
(43) Date of publication of application: **06.12.2023**
(21) Application number: 21934996.6
(22) Date of filing: 01.04.2021
(51) Int. Cl.: A61M 15/06, G16Y 20/40, G16Y 40/30, A24F 40/50

(54) **CONTROL DEVICE, TERMINAL DEVICE, AND INFORMATION PROCESSING METHOD**

(71) Applicant: Japan Tobacco Inc., Tokyo 105-6927 (JP)
(72) Inventor: SERITA, Kazutoshi, Tokyo 130-8603 (JP); SUGANO, Yuka, Tokyo 130-8603 (JP); SENJU, Masatoshi, Tokyo 130-8603 (JP)
(74) Representative: Hoffmann Eitle
(86) International application number: PCT/JP2021/014108
(87) International publication number: WO 2022/208834

(57) **Abstract**

[Problem] To provide a mechanism with which it is possible to improve the quality of the user's puff experience using biometric information.

[Solution] The control device comprises a selection processing unit which selects a second operation setting, which is an operation setting suitable for a user, on the basis of: a first operation setting that is an operation setting according to which a suction device generates an aerosol suctioned by a user; and biometric information on the user corresponding to the first operation setting.

## Description

### Technical Field

The present invention relates to a control device, a terminal device, and an information processing method.

### Background Art

An inhaler device that generates material to be inhaled by a user, such as an electronic tobacco and a nebulizer, is widely used. For example, an inhaler device uses a substrate including an aerosol source for producing an aerosol, a flavor source for imparting a flavor component to the generated aerosol, and the like, to generate an aerosol with the imparted flavor component. The user is able to taste a flavor by inhaling the aerosol with the imparted flavor component, generated by the inhaler device. An action that the user takes to inhale an aerosol is also referred to as puff or puff action below.

In recent years, it has been studied that a sensor that detects biometric information is mounted in an inhaler device. For example, PTL 1 describes a technology for mounting a sensor that detects biometric information in an inhaler device and providing a user with the detected biometric information.

### Citation List

### Patent Literature

Patent Literature 1: WO 2019/175810 A1

### Summary of Invention

### Technical Problem

However, even when biometric information is merely provided to a user, it is difficult to improve the quality of user's puff experience.

The present invention is contemplated in view of the above problem, and it is an object of the present invention to provide a mechanism capable of improving the quality of user's puff experience by using biometric information.

### Solution to Problem

To solve the above problem, according to an aspect of the present invention, a control device is provided. The control device includes a selection processor that selects a second operation setting, which is an operation setting suitable for a user, in accordance with a first operation setting that is an operation setting according to which an inhaler device generates an aerosol to be inhaled by the user and biometric information on the user corresponding to the first operation setting.

The selection processor may estimate a condition of the user in accordance with the biometric information corresponding to the first operation setting and select the second operation setting in accordance with the estimated condition of the user.

The biometric information corresponding to the first operation setting may include the biometric information detected when the inhaler device has generated an aerosol in accordance with the first operation setting.

The biometric information corresponding to the first operation setting may include the biometric information detected before the inhaler device generates an aerosol and after the inhaler device has generated an aerosol in accordance with the first operation setting.

The biometric information corresponding to the first operation setting may be collected when the inhaler device executes a process to generate an aerosol.

The selection processor may select the second operation setting when the inhaler device executes a process to generate an aerosol.

The selection processor may control a process to store, in a memory, information used to select the second operation setting, including a combination of the first operation setting with at least any one of the biometric information corresponding to the first operation setting and a condition of the user estimated in accordance with the biometric information corresponding to the first operation setting.

The selection processor may select the second operation setting in accordance with the information stored in the memory.

The selection processor may select the operation setting, not stored in the memory, as the second operation setting.

The selection processor may select the operation setting, stored in the memory, as the second operation setting.

The selection processor may select the second operation setting further in accordance with information indicating a substrate used by the inhaler device to generate an aerosol.

The selection processor may select the second operation setting further in accordance with information input from the user.

The information input from the user may be an evaluation on the aerosol inhaled by the user.

The information input from the user may be information input to at least any one of the inhaler device, a device carried by the user, and a device worn on the user.

The selection processor may send information indicating the second operation setting.

The biometric information may include at least any one of heart beat, pulse, blood pressure, blood oxygen saturation level, blood volume change, blood flow, respiration, and salivary component.

The biometric information may be detected with at least any one of the inhaler device, a device carried by the user, and a device worn on the user.

The inhaler device may generate the aerosol by heating an aerosol source included in a substrate, and the operation setting may be a setting on temperature to heat the aerosol source.

To solve the above problem, according to another aspect of the present invention, a terminal device is provided. The terminal device includes a controller that controls a process to send a first operation setting that is an operation setting in accordance with which an inhaler device is to generate an aerosol to be inhaled by a user, and biometric information of the user corresponding to the first operation setting, receive information indicating a second operation setting that is the operation setting selected in accordance with the first operation setting and the biometric information corresponding to the first operation setting and suitable for the user, and output the information.

To solve the above problem, according to another aspect of the present invention, an information processing method is provided. The information processing method includes selecting a second operation setting, which is an operation setting suitable for a user, in accordance with a first operation setting that is an operation setting according to which an inhaler device generates an aerosol to be inhaled by the user and biometric information on the user corresponding to the first operation setting.

### Advantageous Effects of Invention

As described above, according to the present invention, a mechanism capable of improving the quality of user's puff experience by using biometric information is provided.

### Brief Description of Drawings

[Fig. 1] Fig. 1 is a schematic diagram that schematically illustrates a first configuration example of an inhaler device.
[Fig. 2] Fig. 2 is a schematic diagram that schematically illustrates a second configuration example of an inhaler device.
[Fig. 3] Fig. 3 is a block diagram that illustrates an example of the configuration of a system according to a present embodiment.
[Fig. 4] Fig. 4 is a sequence diagram that illustrates an example of the flow of a process to recommend an operation setting to be executed by the system according to the present embodiment.

### Description of Embodiments

Hereinafter, a preferred embodiment of the present invention will be described in detail with reference to the attached drawings. In the specification and the drawings, like reference signs denote structural elements having substantially the same functional configuration, and the description will not be repeated.

In the specification and the drawings, elements each having substantially the same functional configuration can be distinguished from one another by suffixing different alphabets to the same reference signs. For example, a plurality of elements each having substantially the same functional configuration is distinguished from one another like inhaler devices 100A, 100B where necessary. However, when a plurality of elements each having substantially the same functional configuration does not need to be distinguished from one another, only the same reference sign is assigned. When, for example, the inhaler devices 100A, 100B do not need to be distinguished from one another, the inhaler device 100A, 100B are simply referred to as inhaler devices 100.

### <1. Configuration Example of Inhaler Device>

An inhaler device is a device that generates material to be inhaled by a user. Hereinafter, the description will be made on the assumption that the material to be generated by the inhaler device is an aerosol. Alternatively, a material to be generated by the inhaler device may be gas.

### (1) First configuration example

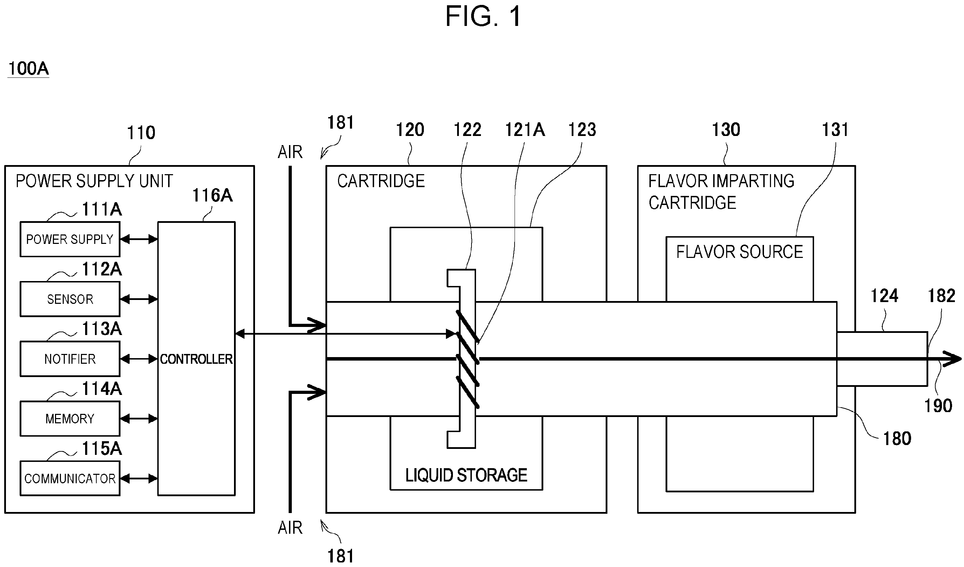

Fig. 1 is a schematic diagram that schematically illustrates a first configuration example of an inhaler device. As illustrated in Fig. 1, an inhaler device 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The power supply 111A stores electric power. The power supply 111A supplies electric power to the structural elements of the inhaler device 100A under the control of the controller 116A. The power supply 111A can be a rechargeable battery, such as a lithium ion secondary battery.

The sensor 112A acquires various items of information regarding the inhaler device 100A. In an example, the sensor 112A may be a pressure sensor such as a capacitor microphone, a flow sensor, or a temperature sensor, and acquire a value generated in accordance with the user's inhalation. In another example, the sensor 112A is an input device that receives information input by the user, such as a button and a switch.

The notifier 113A provides information to the user. The notifier 113A may be a light-emitting device that emits light, a display device that displays an image, a sound output device that outputs sound, or a vibration device that vibrates.

The memory 114A stores various items of information for operation of the inhaler device 100A. The memory 114A is, for example, a non-volatile storage medium, such as a flash memory.

The communicator 115A is a communication interface capable of communication in conformity with any wired or wireless communication standard. For example, Wi-Fi (registered trademark), Bluetooth (registered trademark), or the like can be adopted as such a communication standard.

The controller 116A functions as an arithmetic processing unit and a control unit and controls the overall operations in the inhaler device 100A in accordance with various programs. The controller 116A includes an electronic circuit, such as a central processing unit (CPU) and a microprocessor.

The liquid storage 123 stores an aerosol source. The aerosol source is atomized to generate an aerosol. The aerosol source is, for example, a liquid, such as polyhydric alcohol and water. Examples of the polyhydric alcohol include glycerine and propylene glycol. The aerosol source may include a flavor component that is either derived from tobacco or not derived from tobacco. For the inhaler device 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1, the heater 121A includes a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. Subsequently, the supply of the electric power may be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is on the airflow path 180 at an upstream position (closer to the air inlet hole 181), and the flavor source 131 is on the airflow path 180 at a downstream position (closer to the air outlet hole 182). Air flowing in through the air inlet hole 181 when the user inhales mixes with the aerosol generated by the heater 121A. Subsequently, as indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is a member to be held in a mouth of the user during inhalation. The mouthpiece 124 has the air outlet hole 182. When the user inhales with the mouthpiece 124 in his/her mouth, the mixture fluid of the aerosol and the air enters the oral cavity of the user.

The configuration example of the inhaler device 100A has been described above. The inhaler device 100A is not limited to the above configuration, and may be configured in various ways as exemplified below.

In an example, the inhaler device 100A does not have to include the flavor imparting cartridge 130. In this case, the cartridge 120 includes the mouthpiece 124.

In another example, the inhaler device 100A may include various types of aerosol sources. Still another type of aerosol may be generated by mixing a plurality of types of aerosols generated from the plurality of types of aerosol sources in the airflow path 180 and causing a chemical reaction.

In addition, means for atomizing the aerosol source is not limited to heating by the heater 121A. For example, the means for atomizing the aerosol source may be vibration atomization or induction heating.

### (2) Second configuration example

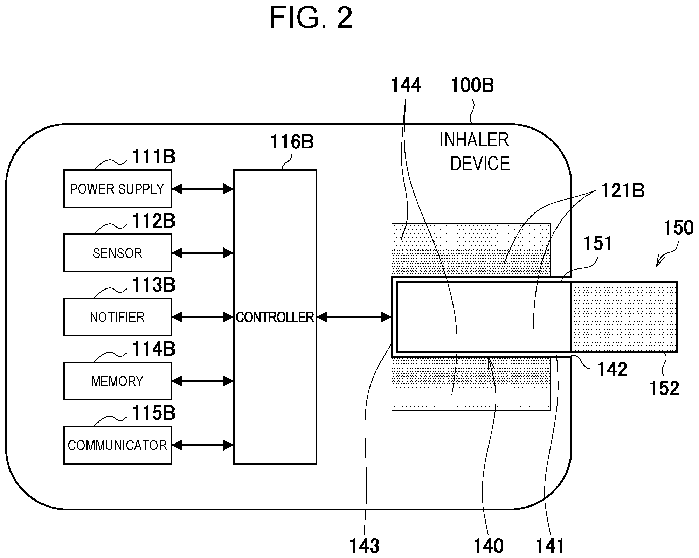

Fig. 2 is a schematic diagram that schematically illustrates a second configuration example of an inhaler device. As illustrated in Fig. 2, an inhaler device 100B according to the present configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the respective corresponding structural elements included in the inhaler device 100A according to the first configuration example.

The holder 140 has an internal space 141. The holder 140 holds the stick substrate 150 while accommodating part of the stick substrate 150 in the internal space 141. The holder 140 has an opening 142 that allows the internal space 141 to communicate with outside. The holder 140 holds the stick substrate 150 that is inserted into the internal space 141 through the opening 142. For example, the holder 140 is a tubular body having the opening 142 and a bottom 143 at its end, and defines the pillar-shaped internal space 141. The holder 140 also has the function to define a flow path for air supplied to the stick substrate 150. An air inlet hole that is an inlet for air into the flow path is disposed at, for example, the bottom 143. On the other hand, an air outlet hole that is an outlet for air from the flow path is the opening 142.

The stick substrate 150 includes a substrate 151 and an inhalation port 152. The substrate 151 includes an aerosol source. In the present configuration example, the aerosol source is not limited to a liquid and may be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 through the air inlet hole (not illustrated), and the air and an aerosol generated from the substrate 151 reach inside the mouth of the user.

The heater 121B has a similar configuration to that of the heater 121A according to the first configuration example. However, in the example illustrated in Fig. 2, the heater 121B has a film-like shape and surrounds the outer circumference of the holder 140. Subsequently, heat produced from the heater 121B heats the substrate 151 of the stick substrate 150 from the outer circumference, generating the aerosol.

The heat insulator 144 prevents heat from transferring from the heater 121B to the other structural elements. For example, the heat insulator 144 may be a vacuum heat insulator or an aerogel heat insulator.

The configuration example of the inhaler device 100B has been described above. The inhaler device 100B is not limited to the above configuration, and may be configured in various ways as exemplified below.

In an example, the heater 121B may have a blade-like shape, and may be disposed so that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed so that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may sandwich the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the inhaler device 100B may also include the heater 121A, the liquid guide 122, the liquid storage 123, and the airflow path 180 according to the first configuration example. The air outlet hole 182 of the airflow path 180 may also serve as an air inlet hole to the internal space 141. In this case, a mixture fluid of the air and an aerosol generated by the heater 121A flows into the internal space 141, mixes further with an aerosol generated by the heater 121B, and then reaches the oral cavity of the user.

### <2. Technical Features>

### <2.1. System Configuration>

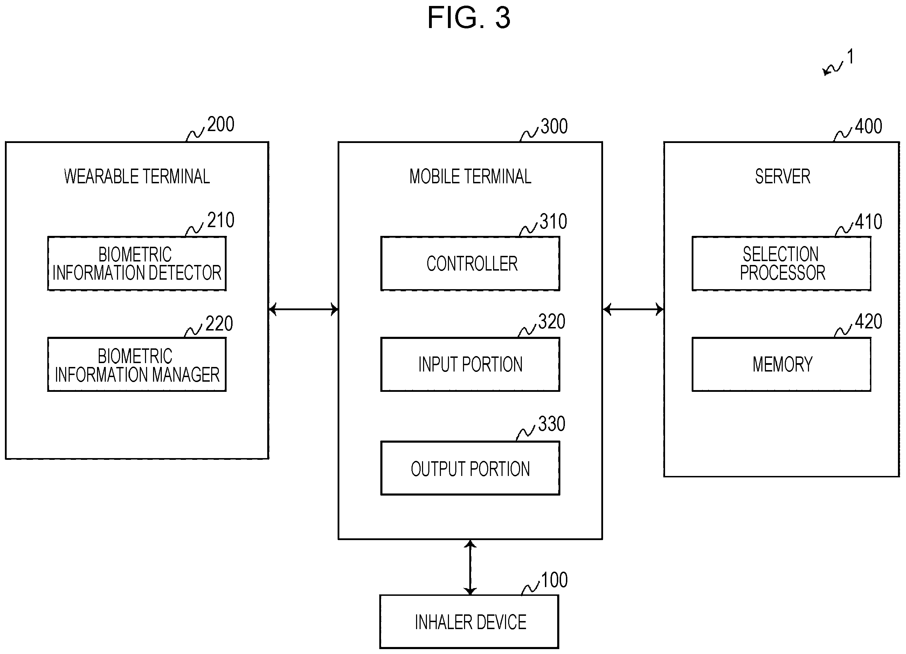

Fig. 3 is a block diagram that illustrates an example of the configuration of a system 1 according to the present embodiment. As illustrated in Fig. 3, the system 1 according to the present embodiment includes the inhaler device 100, a wearable terminal 200, a mobile terminal 300, and a server 400. Fig. 3 illustrates only structural elements particularly related to the present embodiment, and the other structural elements are omitted. For example, each of these devices includes a communicator that communicates with other devices, an input portion that receives information input, an output portion that outputs information, a controller that controls a process over the whole of the device, and the like; however, illustration can be omitted. In other words, each of these devices can include a communication interface, an input device, an output device, and an electronic circuit, such as a CPU.

### (1) Configuration of Inhaler Device 100

The inhaler device 100 can be any configuration example of the first configuration example and the second configuration example described above. The inhaler device 100 according to the present embodiment generates an aerosol to be inhaled by a user by using a substrate. The heater 121 is an example of a generator that generates an aerosol. The cartridge 120 and the flavor imparting cartridge 130 according to the first configuration example and the stick substrate 150 according to the second configuration example are examples of the substrate according to the present invention. The inhaler device 100 generates an aerosol by using a substrate attached or inserted in the inhaler device 100. In the first configuration example, the cartridge 120 and the flavor imparting cartridge 130 connected to the power supply unit 110 are examples of the substrate attached to the inhaler device 100. In the second configuration example, the stick substrate 150 inserted in the inhaler device 100 is an example of the substrate inserted in the inhaler device 100.

The inhaler device 100 generates an aerosol to be inhaled by a user in accordance with an operation setting. When the inhaler device 100 generates an aerosol by heating an aerosol source included in the inhaler device 100, the operation setting is a setting on temperature to heat the aerosol source.

In the first configuration example, the operation setting may include a combination of a target temperature that indicates a temperature for the heater 121 to reach with a heating time that indicates the length of time for the heater 121 to continue heating. In this case, the inhaler device 100 performs heating with the heater 121 such that the temperature of the heater 121 reaches the target temperature in the heating time each time a puff is taken once.

In the second configuration example, the operation setting may include time-series changes in the target temperature that indicates a temperature for the heater 121 to reach. In this case, the inhaler device 100 performs heating with the heater 121 such that time-series changes in the temperature of the heater 121 from the start of heating are similar to time-series changes in the target temperature.

### (2) Wearable Terminal 200

The wearable terminal 200 is a device to be worn by a user. The wearable terminal 200 can have any type, such as a watch type, a necklace type, and an oral cavity attached type. As illustrated in Fig. 3, the wearable terminal 200 includes a biometric information detector 210 and a biometric information manager 220.

The biometric information detector 210 has a function to detect biometric information of a user wearing the wearable terminal 200. The biometric information includes at least any one of heart beat, pulse, blood pressure, blood oxygen saturation level, blood volume change, blood flow, respiration, and salivary component. The biometric information detector 210 includes various sensors for detecting these pieces of biometric information.

The biometric information manager 220 has a function to manage the operation of the biometric information detector 210 and biometric information detected by the biometric information detector 210. For example, the biometric information manager 220 controls the biometric information detector 210 such that the biometric information detector 210 periodically detects biometric information. The biometric information manager 220 stores the detected biometric information detector 210 in association with detection time. After that, the biometric information manager 220 sends the stored biometric information in response to a request from another device.

### (3) Mobile Terminal 300

The mobile terminal 300 is a device to be worn on a user. The mobile terminal 300 can be any type, such as a smartphone and a tablet terminal. The mobile terminal 300 is an example of the terminal device according to the present embodiment. As illustrated in Fig. 3, the mobile terminal 300 includes a controller 310, an input portion 320, and an output portion 330.

The controller 310 is a device that controls a series of processes for recommending an operation setting suitable for the user. For example, the controller 310 collects information to be used to recommend an operation setting and sends the information to a selection processor 410. The controller 310 controls a process to recommend the operation setting selected by the selection processor 410 for the user (for example, a process to cause the output portion 330 to output information that indicates the selected operation setting) in accordance with information received from the selection processor 410.

The input portion 320 has a function to receive information input by the user. The input portion 320 is made up of various input devices, such as a touch panel, a button, and a microphone.

The output portion 330 has a function to output information to the user. The output portion 330 is made up of various output devices, such as a display and a speaker.

### (4) Server 400

The server 400 has a function to execute a process for selecting an operation setting recommended for the user. The server 400 is operated by a company that performs, for example, related business, such as manufacturing and selling the inhaler device 100 or a substrate. The server 400 is an example of the control device according to the present embodiment. As illustrated in Fig. 3, the server 400 includes the selection processor 410 and a memory 420. The selection processor 410 has a function to select an operation setting to be recommended for the user.. The memory 420 has a function to store information to be referenced when the selection processor 410 selects an operation setting to be recommended for the user.

### <2.2. Recommendation of Operation Setting in Accordance with Biometric Information>

The selection processor 410 selects a second operation setting that is an operation setting suitable for the user in accordance with the first operation setting that is an operation setting in accordance with which the inhaler device 100 generates an aerosol and user's biometric information corresponding to the first operation setting. The biometric information corresponding to the first operation setting is biometric information detected when the user inhales the aerosol generated by the inhaler device 100 in accordance with the first operation setting. The time when the user inhales an aerosol here is a concept with a temporal width, such as before the user inhales the aerosol, while the user is inhaling the aerosol, and after the user inhales the aerosol. With the above configuration, it is possible to recommend the second operation setting suitable for user's body in accordance with the influence on biometric information when the user inhales an aerosol. In consideration of a case where the user is hard to be aware of a change in the biometric information of the user him or herself, it is possible to considerably improve the quality of puff experience with the above configuration.

The selection processor 410 may estimate the condition of the user in accordance with biometric information corresponding to the first operation setting and select the second operation setting in accordance with the estimated condition of the user. In an example, the selection processor 410 estimates a degree of relaxation of the user based on biometric information and, when the selection processor 410 estimates that the user is under strong stress, selects an operation setting for increasing a relaxing effect as the second operation setting. An example of the operation setting for increasing a relaxing effect is an operation setting in which the target temperature is low. In another example, the selection processor 410 estimates user's drowsiness in accordance with biometric information and, when the selection processor 410 estimates that the user feels very sleepy, selects an operation setting having an effect to wake up as the second operation setting. An example of the operation setting having an effect to wake up is an operation setting in which the target temperature is high. With the above configuration, it is possible to change user's physical condition toward a better condition.

The biometric information corresponding to the first operation setting may be collected when the inhaler device 100 executes a process to generate an aerosol. For example, when the inhaler device 100 executes a process to generate an aerosol, the controller 310 collects biometric information from the biometric information manager 220 and sends the biometric information to the selection processor 410 in association with the first operation setting. With the above configuration, it is possible to minimize the frequency to collect biometric information, so it is possible to reduce processing load on the devices.

The biometric information corresponding to the first operation setting may include the biometric information detected when the inhaler device 100 has generated an aerosol in accordance with the first operation setting. For example, at the time when the inhaler device 100 executes a process to generate an aerosol, the controller 310 collects biometric information detected by the biometric information detector 210 and sends the biometric information to the selection processor 410 in association with the first operation setting. When a plurality of pieces of biometric information is detected while the inhaler device 100 is generating an aerosol, the controller 310 may collect the plurality of pieces of detected biometric information. Alternatively, at the time when the inhaler device 100 executes a process to generate an aerosol, the controller 310 may issue a request to detect biometric information, detect biometric information at that time, and acquire the biometric information. With the above configuration, it is possible to recommend the second operation setting in accordance with biometric information that reflects the influence from an inhaled aerosol.

The biometric information corresponding to the first operation setting may include biometric information detected before and after the inhaler device 100 generates an aerosol in accordance with the first operation setting. For example, the controller 310 collects biometric information detected a predetermined time or longer before generation of an aerosol is started and biometric information detected after a lapse of a predetermined time or longer from the end of generation of an aerosol and sends the pieces of biometric information to the selection processor 410 in association with the first operation setting. In this case, the selection processor 410 estimates a change in the condition of the user before and after inhaling an aerosol in accordance with a difference between these pieces of biometric information and selects an operation setting with which a more desirable change in condition is expected as the second operation setting. With the above configuration, it is possible to recommend an operation setting according to a change in the condition of the user before and after inhaling an aerosol as the second operation setting.

The selection processor 410 may select the second operation setting when the inhaler device 100 executes a process to generate an aerosol. For example, the controller 310 collects biometric information corresponding to the first operation setting when the inhaler device 100 executes a process to generate an aerosol and sends the collected biometric information and the first operation setting to the selection processor 410. The selection processor 410 selects the second operation setting when the selection processor 410 receives the first operation setting and the biometric information from the controller 310. With the above configuration, the user is able to receive recommendation on the second operation setting immediately after taking a puff.

The selection processor 410 may select the second operation setting in accordance with the latest information. In addition, the selection processor 410 may select the second operation setting in accordance with past information in addition to or instead of the latest information. In this regard, the description will be made below.

The selection processor 410 may control a process to store, in the memory 420, information used to select the second operation setting. In other words, the memory 420 may store information used to select the second operation setting. The information used to select the second operation setting includes a combination of the first operation setting with at least any one of biometric information corresponding to the first operation setting and the condition of the user estimated in accordance with the biometric information corresponding to the first operation setting. With the above configuration, the memory 420 can accumulate a history of the biometric information of the user or the condition of the user at the time of inhaling an aerosol generated in accordance with the first operation setting, in association with the first operation setting.

The selection processor 410 may select the second operation setting in accordance with the information stored in the memory 420. In other words, the selection processor 410 may select the second operation setting by referencing the history of the biometric information of the user or the history of the condition of the user at the time when the user inhales an aerosol generated in accordance with the first operation setting. The biometric information of the user or the condition of the user is presumably influenced by not only an aerosol inhaled but also an external factor, such as an activity made before or after then and a place. In this regard, with the above configuration, since pieces of information collected and accumulated multiple times in the past can be referenced, influence from an external factor is uniformed, so it is possible to recommend a further appropriate second operation setting.

The selection processor 410 may select an operation setting not stored in the memory 420, as the second operation setting. In other words, the selection processor 410 may select an operation setting not yet tried by the user, as the second operation setting. With the above configuration, the user is able to try a new operation setting.

The selection processor 410 may select an operation setting stored in the memory 420, as the second operation setting. In other words, the selection processor 410 may select an operation setting tried in the past by the user, as the second operation setting. For example, the selection processor 410 selects an operation setting used when the user is in a good condition, as the second operation setting. With the above configuration, the user is able to rediscover the merit of the operation setting tried in the past.

### <2.3. Recommendation of Operation Setting in Accordance with Other Information>

### Information Indicating Substrate

The selection processor 410 may select the second operation setting further in accordance with information indicating a substrate used for the inhaler device 100 to generate an aerosol to be inhaled by the user. An example of the information indicating a substrate is information indicating the type (for example, brand) of substrate. For example, the selection processor 410 selects the second operation setting by referencing biometric information collected at the time when the substrate of the same brand as the substrate currently being used by the inhaler device 100 was used in the past. The biometric information of the user is presumably also influenced from a substrate in addition to the first operation setting. In this regard, with the above configuration, it is possible to recommend a second operation setting suitable for the user for each substrate.

The information indicating the type of substrate can be, for example, detected by the sensor 112 at the time when the substrate is attached or inserted in the inhaler device 100. In an example, the type of substrate may be detected by image recognition of identification information attached to the surface of the substrate. In another example, the type of substrate may be detected in accordance with a resistance value when current is applied to the substrate.

The memory 420 may store information indicating a substrate used by the inhaler device 100 to generate an aerosol as information used to select the second operation setting.

### Information Input from User

The selection processor 410 may select the second operation setting further in accordance with information input from the user. In an example, the input portion 320 may receive an evaluation input for an aerosol inhaled by the user immediately after the user inhales the aerosol generated in accordance with the first operation setting. For example, a smoking feeling is input by 5-grade evaluation. The selection processor 410 may select the second operation setting in accordance with an evaluation on an aerosol inhaled by the user. With the above configuration, it is possible to recommend a second operation setting in accordance with a direct evaluation from the user related to the first operation setting.

The memory 420 may store information input from the user as information used to select the second operation setting.

### Time of Puff

The selection processor 410 may select the second operation setting further in accordance with time at which the inhaler device 100 executed a process to generate an aerosol in accordance with the first operation setting, that is, time at which the user took a puff. For example, the selection processor 410 selects the second operation setting by referencing biometric information collected at the time when the user took a puff in the same time period as time at which the user took a puff this time. The biometric information of the user is presumably also influenced from a time period in which a puff is taken in addition to the first operation setting. In this regard, with the above configuration, it is possible to recommend the second operation setting suitable for the user for each time period in which a puff is taken.

The memory 420 may store time at which the inhaler device 100 executes a process to generate an aerosol in accordance with the first operation setting as information used to select the second operation setting.

### <2.4. Information Output>

The selection processor 410 sends information indicating the second operation setting. The information indicating the second operation setting may be the second operation setting itself or may be a difference between the first operation setting and the second operation setting.

In an example, the selection processor 410 may send information indicating the selected second operation setting to the controller 310. The controller 310 outputs the received information indicating the second operation setting to the user by the output portion 330. With the above configuration, the user is able to receive recommendation on the second operation setting. It is possible to provide a motivation to the user to switch into the recommended second operation setting.

In another example, the selection processor 410 may send information indicating the selected second operation setting to the inhaler device 100 via the controller 310. With the above configuration, the inhaler device 100 can switch the operation setting from the first operation setting to the second operation setting and use the second operation setting at the time of generating an aerosol next time. When permission from the user is obtained, information indicating the second operation setting may be sent to the inhaler device 100.

The output portion 330 may output biometric information corresponding to the first operation setting or the condition of the user estimated in accordance with the biometric information corresponding to the first operation setting, in addition to or instead of information indicating the second operation setting. With the above configuration, the user is able to learn grounds that the second operation setting is recommended. Therefore, it is possible to provide a further strong motivation to the user to switch into the recommended second operation setting.

### <2.5. Flow of Process>

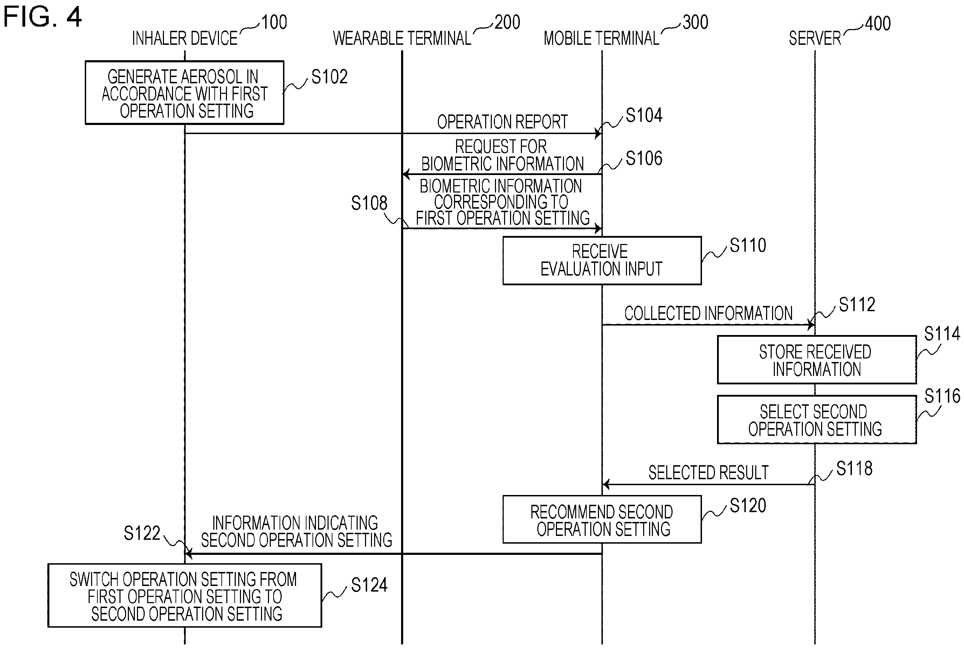

Fig. 4 is a sequence diagram that illustrates an example of the flow of a process to recommend an operation setting to be executed by the system 1 according to the present embodiment. As illustrated in Fig. 4, the inhaler device 100, the wearable terminal 200, the mobile terminal 300, and the server 400 are involved in the sequence. Although not illustrated in Fig. 4, the wearable terminal 200 periodically detects biometric information.

Initially, the inhaler device 100 executes a process to generate an aerosol in accordance with the first operation setting (step S102). Before and after then, the inhaler device 100 identifies the substrate used at the time of generating an aerosol.

Subsequently, the inhaler device 100 sends an operation report to the mobile terminal 300 (step S104). The operation report includes, for example, information indicating time at which a process to generate an aerosol is executed, information indicating the first operation setting, and information indicating a substrate used at the time of generating an aerosol. The inhaler device 100, for example, may send an operation report at the start of heating or may send an operation report after the end of heating.

After that, the mobile terminal 300 sends, to the wearable terminal 200, a request to send biometric information corresponding to the first operation setting (step S106). The request for biometric information includes, for example, information indicating time at which a process to generate an aerosol is executed.

Subsequently, the wearable terminal 200 sends, to the mobile terminal 300, the biometric information corresponding to the first operation setting (step S108). For example, the wearable terminal 200 sends, to the mobile terminal 300, biometric information detected in a period from a predetermined time or longer before to a lapse of a predetermined time or longer after time at which a process to generate an aerosol in accordance with the first operation setting is executed.

Then, the mobile terminal 300 receives from the user an evaluation input for the aerosol inhaled (step S110).

Subsequently, the mobile terminal 300 sends the collected information to the server 400 (step S112). For example, the mobile terminal 300 sends, to the server 400, the information indicating the first operation setting, the biometric information corresponding to the first operation setting, the information indicating the substrate, the evaluation input from the user, and the information indicating time at which a process to generate an aerosol in accordance with the first operation setting is executed.

Subsequently, the server 400 stores the received information as information to be used to select the second operation setting (step S114). The server 400 selects the second operation setting in accordance with the stored information (step S116). For example, the server 400 estimates the condition of the user in accordance with the biometric information corresponding to the first operation setting and selects the second operation setting in accordance with the estimated condition of the user and the first operation setting. In selection, the server 400 may make reference to the type of substrate, the evaluation input from the user, and time at which a process to generate an aerosol is executed.

Subsequently, the server 400 sends, to the mobile terminal 300, information indicating the selected result (step S118). The information indicating the selected result includes, for example, information indicating the selected second operation setting, the biometric information corresponding to the first operation setting, and the information indicating the condition of the user estimated in accordance with the biometric information corresponding to the first operation setting.

After that, the mobile terminal 300 recommends the second operation setting for the user (step S120). For example, the mobile terminal 300 displays the information indicating the second operation setting. In addition, the mobile terminal 300 may output the biometric information corresponding to the first operation setting or the information indicating the condition of the user estimated in accordance with the biometric information corresponding to the first operation setting.

Subsequently, when permission to switch into the second operation setting is input from the user, the mobile terminal 300 sends, to the inhaler device 100, the information indicating the second operation setting (step S122).

Then, the inhaler device 100 switches the operation setting from the first operation setting to the second operation setting (step S124).

### <3. Supplement>

The preferred embodiment of the present invention has been described in detail with reference to the attached drawings; however, the present invention is not limited to those examples. It is obvious that persons having ordinary skill in the art in the field of technology to which the present invention belongs can conceive of various modifications or alterations within the scope of the technical idea recited in the claims, and these can also be naturally interpreted as belonging to the technical scope of the present invention.

For example, the system 1 just has to include the biometric information detector 210, the biometric information manager 220, the controller 310, the input portion 320, the output portion 330, the selection processor 410, and the memory 420 in addition to the inhaler device 100. Which devices these structural elements are mounted on are selectable. One structural element may be mounted on a plurality of devices.

In an example, the biometric information detector 210 and the biometric information manager 220 may be mounted on at least any one of the inhaler device 100, the wearable terminal 200, and the mobile terminal 300. In other words, biometric information may be detected by at least any one of the inhaler device 100, the wearable terminal 200, and the mobile terminal 300. With the above configuration, when, for example, detectable biometric information varies for each device, it is possible to recommend the second operation setting in accordance with a further larger amount of biometric information.

In another example, the input portion 320 may be mounted on at least any one of the inhaler device 100, the wearable terminal 200, and the mobile terminal 300. In other words, the selection processor 410 may select the second operation setting in accordance with information input to at least any one of the inhaler device 100, the wearable terminal 200, and the mobile terminal 300. With the above configuration, when, for example, inputtable information varies for each device, it is possible to recommend the second operation setting in accordance with a further larger amount of input information.

In another example, the output portion 330 may be mounted on at least any one of the inhaler device 100, the wearable terminal 200, and the mobile terminal 300. In other words, the information indicating the second operation setting may be output from at least any one of the inhaler device 100, the wearable terminal 200, and the mobile terminal 300. With the above configuration, for example, it is possible to recommend the second operation setting via a device from which the user easily finds notification.

In another example, the system 1 just has to include at least the inhaler device 100 as a device, and the other devices are not limited. For example, the controller 310, the input portion 320, and the output portion 330 may be mounted on the wearable terminal 200, and the mobile terminal 300 may be omitted from the system 1. In another example, the selection processor 410 and the memory 420 may be mounted on the mobile terminal 300, and the server 400 may be omitted from the system 1.

In another example, in the above embodiment, an example in which a device that includes the controller 310, the input portion 320, and the output portion 330 is the mobile terminal 300 has been described; however, the present invention is not limited to the above example. The controller 310, the input portion 320, and the output portion 330 only have to be devices present near the user when the user uses the inhaler device 100. A digital signage provided in a smoking area is listed as an example of such a device. In this case, when the user takes a puff with the inhaler device 100 in a smoking area, the second operation setting suitable for the user is recommended via the digital signage.

For example, in the above embodiment, an example in which the second operation setting is recommended when the inhaler device 100 executes a process to generate an aerosol, that is, when a puff is taken, has been described; however, the present invention is not limited to this example. In an example, when substrates are sold in units of box in which a plurality of substrates is contained, the second operation setting may be recommended when it is determined from a use history that it is time to finish smoking for a box of substrates. Thus, it is possible to provide a motivation to switch into the second operation setting from the timing to use a substrate purchased next. In another example, the second operation setting may be recommended when the user inputs an evaluation on an aerosol inhaled by the user. In another example, the second operation setting may be recommended when it is determined in accordance with location information that the user approaches a sales office for a substrate. In this case, it is desirable to recommend the second operation setting adaptive to a substrate sold at a shop.

For example, in the above embodiment, an example in which the selection processor 410 selects the second operation setting in accordance with information collected in relation to the user who uses the inhaler device 100 has been described; however, the present invention is not limited to the above example. For example, the selection processor 410 may select the second operation setting to be recommended for the user in accordance with information collected in relation to another user other than the user who uses the inhaler device 100. For example, the selection processor 410 may select the second operation setting to be recommended for the user in accordance with information collected in relation to another user who resembles the user in the tendency of biometric information. With the above configuration, it is possible to further improve recommendation accuracy.

Pieces of processing described by using the flowchart and the sequence diagram in the specification may be not necessarily executed in order as illustrated. Some processing steps may be executed in parallel. An additional processing step may be adopted, and one or some of the processing steps may be omitted.

A series of pieces of processing, executed by the devices, described in the specification, may be implemented by any one of software, hardware, and a combination of software and hardware. Programs that are components of software are prestored in, for example, storage media (non-transitory media) provided inside or outside the devices. The programs are, for example, loaded onto a RAM when a computer that controls the devices described in the specification runs the programs and are run on a processor, such as a CPU. Examples of the storage media include a magnetic disk, an optical disk, a magneto-optical disk, and a flash memory. The computer programs may be distributed via, for example, a network, without using storage media.

The following configurations also belong to the technical scope of the present invention.
(1) A control device includes
   a selection processor that selects a second operation setting, which is an operation setting suitable for a user, in accordance with a first operation setting that is an operation setting according to which an inhaler device generates an aerosol to be inhaled by the user and biometric information on the user corresponding to the first operation setting.
(2) In the control device according to (1),
   the selection processor estimates a condition of the user in accordance with the biometric information corresponding to the first operation setting and selects the second operation setting in accordance with the estimated condition of the user.
(3) In the control device according to (1) or (2),
   the biometric information corresponding to the first operation setting includes the biometric information detected when the inhaler device has generated an aerosol in accordance with the first operation setting.
(4) In the control device according to any one of (1) to (3),
   the biometric information corresponding to the first operation setting includes the biometric information detected before the inhaler device generates an aerosol and after the inhaler device has generated an aerosol in accordance with the first operation setting.
(5) In the control device according to any one of (1) to (4),
   the biometric information corresponding to the first operation setting is collected when the inhaler device executes a process to generate an aerosol.
(6) In the control device according to any one of (1) to (5),
   the selection processor selects the second operation setting when the inhaler device executes a process to generate an aerosol.
(7) In the control device according to any one of (1) to (6),
   the selection processor controls a process to store, in a memory, information used to select the second operation setting, including a combination of the first operation setting with at least any one of the biometric information corresponding to the first operation setting and a condition of the user estimated in accordance with the biometric information corresponding to the first operation setting.
(8) In the control device according to (7),
   the selection processor selects the second operation setting in accordance with the information stored in the memory.
(9) In the control device according to (8),
   the selection processor selects the operation setting, not stored in the memory, as the second operation setting.
(10) In the control device according to (8),
   the selection processor selects the operation setting, stored in the memory, as the second operation setting.
(11) In the control device according to any one of (1) to (10),
   the selection processor selects the second operation setting further in accordance with information indicating a substrate used by the inhaler device to generate an aerosol.
(12) In the control device according to any one of (1) to (11),
   the selection processor selects the second operation setting further in accordance with information input from the user.
(13) In the control device according to (12),
   the information input from the user is an evaluation on the aerosol inhaled by the user.
(14) In the control device according to (12) or (13),
   the information input from the user is information input to at least any one of the inhaler device, a device carried by the user, and a device worn on the user.
(15) In the control device according to any one of (1) to (14),
   the selection processor sends information indicating the second operation setting.
(16) In the control device according to any one of (1) to (15),
   the biometric information includes at least any one of heart beat, pulse, blood pressure, blood oxygen saturation level, blood volume change, blood flow, respiration, and salivary component.
(17) In the control device according to any one of (1) to (16),
   the biometric information is detected with at least any one of the inhaler device, a device carried by the user, and a device worn on the user.
(18) In the control device according to any one of (1) to (17),
   the inhaler device generates the aerosol by heating an aerosol source included in a substrate, and
   the operation setting is a setting on temperature to heat the aerosol source.
(19) A terminal device includes
   a controller that controls a process to send a first operation setting that is an operation setting in accordance with which an inhaler device is to generate an aerosol to be inhaled by a user, and biometric information of the user corresponding to the first operation setting, receive information indicating a second operation setting that is the operation setting selected in accordance with the first operation setting and the biometric information corresponding to the first operation setting and suitable for the user, and output the information.
(20) An information processing method includes
   selecting a second operation setting, which is an operation setting suitable for a user, in accordance with a first operation setting that is an operation setting according to which an inhaler device generates an aerosol to be inhaled by the user and biometric information on the user corresponding to the first operation setting.

### Reference Signs List

- 1: system
- 100: inhaler device
- 111: power supply
- 112: sensor
- 113: notifier
- 114: memory
- 115: communicator
- 116: controller
- 121: heater
- 140: holder
- 141: internal space
- 142: opening
- 143: bottom
- 144: heat insulator
- 150: stick substrate
- 151: substrate
- 152: inhalation port
- 200: wearable terminal
- 210: biometric information detector
- 220: biometric information manager
- 300: mobile terminal
- 310: controller
- 320: input portion
- 330: output portion
- 400: server
- 410: selection processor
- 420: memory

## Claims

1. A control device comprising:
a selection processor that selects a second operation setting, which is an operation setting suitable for a user, in accordance with a first operation setting that is an operation setting according to which an inhaler device generates an aerosol to be inhaled by the user and biometric information on the user corresponding to the first operation setting.

2. The control device according to claim 1, wherein
the selection processor estimates a condition of the user in accordance with the biometric information corresponding to the first operation setting and selects the second operation setting in accordance with the estimated condition of the user.

3. The control device according to claim 1 or 2, wherein
the biometric information corresponding to the first operation setting includes the biometric information detected when the inhaler device has generated an aerosol in accordance with the first operation setting.

4. The control device according to any one of claims 1 to 3, wherein
the biometric information corresponding to the first operation setting includes the biometric information detected before the inhaler device generates an aerosol and after the inhaler device has generated an aerosol in accordance with the first operation setting.

5. The control device according to any one of claims 1 to 4, wherein
the biometric information corresponding to the first operation setting is collected when the inhaler device executes a process to generate an aerosol.

6. The control device according to any one of claims 1 to 5, wherein
the selection processor selects the second operation setting when the inhaler device executes a process to generate an aerosol.

7. The control device according to any one of claims 1 to 6, wherein
the selection processor controls a process to store, in a memory, information used to select the second operation setting, including a combination of the first operation setting with at least any one of the biometric information corresponding to the first operation setting and a condition of the user estimated in accordance with the biometric information corresponding to the first operation setting.

8. The control device according to claim 7, wherein
the selection processor selects the second operation setting in accordance with the information stored in the memory.

9. The control device according to claim 8, wherein
the selection processor selects the operation setting, not stored in the memory, as the second operation setting.

10. The control device according to claim 8, wherein
the selection processor selects the operation setting, stored in the memory, as the second operation setting.

11. The control device according to any one of claims 1 to 10, wherein
the selection processor selects the second operation setting further in accordance with information indicating a substrate used by the inhaler device to generate an aerosol.

12. The control device according to any one of claims 1 to 11, wherein
the selection processor selects the second operation setting further in accordance with information input from the user.

13. The control device according to claim 12, wherein
the information input from the user is an evaluation on the aerosol inhaled by the user.

14. The control device according to claim 12 or 13, wherein
the information input from the user is information input to at least any one of the inhaler device, a device carried by the user, and a device worn on the user.

15. The control device according to any one of claims 1 to 14, wherein
the selection processor sends information indicating the second operation setting.

16. The control device according to any one of claims 1 to 15, wherein
the biometric information includes at least any one of heart beat, pulse, blood pressure, blood oxygen saturation level, blood volume change, blood flow, respiration, and salivary component.

17. The control device according to any one of claims 1 to 16, wherein
the biometric information is detected with at least any one of the inhaler device, a device carried by the user, and a device worn on the user.

18. The control device according to any one of claims 1 to 17, wherein
the inhaler device generates the aerosol by heating an aerosol source included in a substrate, and
the operation setting is a setting on temperature to heat the aerosol source.

19. A terminal device comprising:
a controller that controls a process to send a first operation setting that is an operation setting in accordance with which an inhaler device is to generate an aerosol to be inhaled by a user, and biometric information of the user corresponding to the first operation setting, receive information indicating a second operation setting that is the operation setting selected in accordance with the first operation setting and the biometric information corresponding to the first operation setting and suitable for the user, and output the information.

20. An information processing method comprising
selecting a second operation setting, which is an operation setting suitable for a user, in accordance with a first operation setting that is an operation setting according to which an inhaler device generates an aerosol to be inhaled by the user and biometric information on the user corresponding to the first operation setting.
